# EUROPEAN PATENT APPLICATION

(11) **EP 2 018 868 A1**
(43) Date of publication of application: **28.01.2009**
(21) Application number: 08013227.7
(22) Date of filing: 23.07.2008
(51) Int. Cl.: A61K 36/16, A61K 31/352, A61K 31/7068

(54) **Compositions for the treatment and prevention of vertigo and tinnitus including citicoline, ginkgo biloba extract and dimeric flavones of ginkgo biloba**

(30) Priority: 24.07.2007 IT MI20071492
(71) Applicant: S.I.I.T. S.r.l. Servizio Internazionale Imballaggi Termosaldanti, 20090 Trezzano S/N (Milan) (IT)
(72) Inventor: Di Pierro, Francesco, 20090 Trezzano S/N Milano (IT)
(74) Representative: Minoja, Fabrizio

(57) **Abstract**

Disclosed are compositions containing:
a) citicoline
*b) Ginkgo biloba* extract; and
c) dimeric flavones of *Ginkgo biloba;*
for the treatment and prevention of vertigo and tinnitus.

## Description

The present invention relates to compositions containing citicoline, *Ginkgo biloba* extract and dimeric flavones of *Ginkgo biloba* for the treatment and prevention of vertigo and tinnitus.

The invention also relates to the use of a combination of citicoline, *Ginkgo biloba* extract and dimeric flavones of *Ginkgo biloba* for the preparation of a formulation for the treatment and prevention of vertigo and tinnitus.

### PRIOR ART

Vertigo or dizziness is an often debilitating condition that causes sensations of loss of balance and illusory impressions of movement of the sufferer's body or surroundings.

The causes of vertigo may be otogenic (as in the case of Ménière's syndrome), ie. associated with imbalances of the otovestibular apparatus and labyrinth, toxic (e.g. caused by drugs or alcohol), psychogenic, circulatory, neurological, neoplastic, haematogenous or ocular (diplopia).

The treatment of vertigo obviously depends on its etiology, but symptomatic treatment is currently based on drugs such as dimenhydrinate, perphenazine and meclizine.

Tinnitus (from the Latin "tinnire", which means "ringing") consists of a perception, in one or both ears, of a sound not deriving from the external environment, which may be present continuously or intermittently in the form of buzzing, whistling, ringing, the sound of waterfalls, bells and so on, generally of an acute pitch.

Tinnitus is usually only perceived by the sufferer, although in some cases, when it is caused by disorders associated with vascular problems due to alterations in the blood flow in the ear, an external observer may also perceive it.

Tinnitus can have various origins: it may be otogenic (caused by accumulation of wax, reduction of hearing capacity, inflammatory processes of the middle and inner ear, bacterial infections, and labyrinthitis), physiological (alterations of the circulation, hypertension) or caused by taking drugs like aspirin, streptomycin and other antibiotics.

When the cause of tinnitus is a reduction in hearing capacity, hearing aids often worsen the patient's condition.

The severity of tinnitus can range from slight discomfort to a serious condition that adversely affects the patient's quality of life.

Although laser treatments have been proposed to regenerate the damaged cells, as have pharmacological treatments with *Ginkgo biloba* extracts (Plath P, Olivier J., Adv Otorhinolaryngol. 1995; 49:101-4), ambroxol (WO2005007147), GABA analogues (WO03063845) and substituted aminoalkanesulphonic acids (WO03092701), no really effective treatment for tinnitus has yet been found.

It has now been found that a combination of citicoline with a *Ginkgo biloba* extract and dimeric flavones of *Ginkgo biloba* provides an effective remedy substantially devoid of side effects, for the treatment and prevention of vertigo and tinnitus of various etiologies.

Citicolinè or citidin-5'-diphosphocholine (CDP-choline) is a drug which has long been used parenterally for the treatment of cerebrovascular disorders with a traumatic or degenerative origin, impaired consciousness, cerebral atherosclerosis, and the sequelae of haemorrhages, strokes, thrombosis and Parkinson's disease.

*Ginkgo biloba* extracts have been known and used for some time in phytotherapy and as diet supplements due to their neurostimulating and energising properties. Various methods are known for the extraction of the vegetable material, usually the leaves. See, for example, EP 324197, EP 86315 and CH 674365. Compounds called ginkgoflavon glycosides (a content of not less than 24%), bilobalide and ginkgolides (the last 2 must have a total content of not less than 6%) have been identified among the main active constituents of the extract. Complexes of bilobalide and ginkgolides with phospholipids were described in EP 441279 as neurotrophic agents, anti-inflammatories and PAF-antagonists.

Dimeric flavones of *Ginkgo biloba* are a particular molecular fraction, not contained in the standardised extract of *Ginkgo biloba* described above, which are obtained by a specific extraction process from the leaves of *Ginkgo biloba.* This fraction corresponds to a highly purified mixture of five non-glycosylated biflavonic structures: amentoflavone, bilobetin, ginkgetin, isoginkgetin and sciadopitysin. These compounds are widely used in the cosmetics field in the treatment of cellulite and in antiaging skin treatments due to their specific antioxidant action towards nitric oxide (NO). Nitric oxide, produced enzymatically by the epithelial and inflammatory cells found in the tissue, possesses considerable vasodilating properties. However, NO has a half-life of a few seconds, and is subsequently deactivated by the free radicals in the environment. The action of the fraction in question therefore seems to be to prolong the half-life of NO by acting "from the outside", ie. defending it against oxidative stress with antioxidant substances which have demonstrated a high degree of specificity for that molecule.

Biflavones of *Ginkgo biloba* also inhibit *in vitro* the release of histamine, one of the main mediators of states of skin irritation, by the mast cells; they perform a good anti-inflammatory action, and are powerful inhibitors of phosphodiesterases, enzymes involved in the metabolism of the cyclic nucleotides.

It has now surprisingly been observed that the addition of this third ingredient, well known mainly in the cosmetics industry, considerably improves the clinical symptoms associated with vertigo and tinnitus. The speed and intensity of action of the formulation are also unexpected in view of the efficacy, already demonstrated in the past and described in Italian patent application (M12004A001078), of the formula developed in the absence of this dimeric fraction by G. biloba in the form of complexes with phospholipids (phytosomes®).

### DESCRIPTION OF THE INVENTION

It has now been discovered that oral administration of compositions containing citicoline, extracts of *Ginkgo biloba* leaves and dimeric flavones of *Ginkgo biloba* eliminate or drastically reduce vertigo and tinnitus.

This invention therefore relates to compositions containing:
a) citicoline;
b) *Ginkgo biloba* extract; and
c) dimeric flavones of *Ginkgo biloba;*
for the treatment and prevention of vertigo and tinnitus.

According to a preferred aspect of this invention, the *Ginkgo biloba* extract will be an extract of *Ginkgo biloba* leaves, and even more preferably a dried extract of *Ginkgo biloba* leaves.

According to a further preferred aspect of this invention, the *Ginkgo biloba* extract and the dimeric flavones of *Ginkgo biloba* will be present in the compositions in complexed form with phospholipids, as marketed under the name of Phytosome®.

The doses of the basic ingredients of the association will fall into in the following weight intervals:
a) citicoline: between 10 and 1000 mg per unit dose;
b) dried extract of *Ginkgo biloba* or corresponding complex with phospholipids: between 10 and 360 mg per unit dose; and
c) dimeric flavones of *Ginkgo biloba* or corresponding complex with phospholipids: between 5 and 150 mg per unit dose.

According to a preferred aspect of this invention, the doses of the basic ingredients of the association will fall into the following weight intervals:
a) citicoline: between 100 and 400 mg per unit dose;
b) dried extract of *Ginkgo biloba* or corresponding complex with phospholipids: between 40 and 120 mg per unit dose; and
c) dimeric flavones of *Ginkgo biloba* or corresponding complex with phospholipids: between 10 and 60 mg per unit dose.

According to a preferred aspect of this invention, the compositions in question can contain additional ingredients with a curative or complementary action, or otherwise useful for the purposes of the invention. Examples of these additional ingredients are vitamins, aminoacids and trace elements, such as L-arginine (or any other aminoacid or aminoacid-like substance able to promote the synthesis, release or metabolism of nitric oxide), selenium, zinc, magnesium, vitamin A, vitamin E, and Group B vitamins (B1, B2, B6 and B12).

These additional ingredients will be present in amounts corresponding to their Recommended Dietary Allowance (or multiples or sub-multiples thereof), according to the intervals described here by way of example:
- L-Arginine 10-1000 mg
- Selenium 0.001-10 mg
- Zinc 0.001-10 mg
- Magnesium 0.001 - 10 mg
- Vitamin A 1-10,000 I
- Vitamin E 0.1-60 mg
- Group B vitamins 0.001-20 mg.

The compositions according to the invention can be formulated in any form suitable for oral administration, such as hard or soft gelatin capsules, tablets, effervescent or chewable tablets, granules or powders in sachets, controlled-release solid forms, chewing gums and the like.

The compositions according to the invention can be formulated suitably for oral administration, and prepared according to conventional methods well known in pharmaceutical technology, such as those described in Remington's Pharmaceutical Handbook, Mack Publishing Co., N.Y., USA, using excipients, diluents, fillers and anti-caking agents acceptable for their final use.

The following examples illustrate the invention in greater detail.

### EXAMPLE 1 - 3 g sachets

| **INGREDIENT** | **mg/unit dose** | **%** |
|---|---|---|
| Citicoline | 400.000 | 13.201 |
| *Ginkgo biloba* dried extract | 100.000 | 3.300 |
| *Ginkgo biloba* dimeric flavones | 30.000 | 0.990 |
| Saccharose | 2265.000 | 74.752 |
| Citric acid | 50.000 | 1.650 |
| Silicon dioxide | 20.000 | 0.660 |
| Flavouring | 150.000 | 4.950 |
| Acesulfame K | 15.000 | 0.495 |
| **TOTAL** | **3030.000** | |

### EXAMPLE 2 - 3 g sachets

| **INGREDIENT** | **mg/unit dose** | **%** |
|---|---|---|
| Citicoline | 400.000 | 13.333 |
| *Ginkgo biloba* dried extract | 100.000 | 3.333 |
| *Ginkgo biloba* dimeric flavones | 30.000 | 1.000 |
| Isomalt | 2220.000 | 74.000 |
| Citric acid | 50.000 | 1.667 |
| Silicon dioxide | 20.000 | 0.667 |
| Flavouring | 150.000 | 5.000 |
| Acesulfame K | 15.000 | 0.500 |
| Aspartame | 15.000 | 0.500 |
| | | |
| **TOTAL** | **3000.000** | |

### EXAMPLE 3 - 3 g sachets

| **INGREDIENT** | **mg/unit dose** | **%** |
|---|---|---|
| Citicoline | 400.000 | 13.333 |
| *Ginkgo biloba* dried extract phytosome | 100.000 | 3.333 |
| *Ginkgo biloba* dimeric flavones phytosome | 30.000 | 1.000 |
| Saccharose | 2235.000 | 74.500 |
| Citric acid | 50.000 | 1.667 |
| Silicon dioxide | 20.000 | 0.667 |
| Flavouring | 150.000 | 5.000 |
| Acesulfame K | 15.000 | 0.500 |
| | | |
| **TOTAL** | **3000.00C** | |

### EXAMPLE 4 - 3 g sachets

| **INGREDIENT** | **mg/unit dose** | **%** |
|---|---|---|
| Citicoline | 400.000 | 13.333 |
| *Ginkgo biloba* dried extract phytosome | 100.000 | 3.333 |
| *Ginkgo biloba* dimeric flavones phytosome | 30.000 | 1.000 |
| Isomalt | 2220.000 | 74.000 |
| Citric acid | 50.000 | 1.667 |
| Silicon dioxide | 20.000 | 0.667 |
| Flavouring | 150.000 | 5.000 |
| Acesulfame K | 15.000 | 0.500 |
| Aspartame | 15.000 | 0.500 |
| | | |
| **TOTAL** | **3000.000** | |

### EXAMPLE 5 - 0+ format 610 mg capsules

| **INGREDIENT** | **mg/unit dose** | **%** |
|---|---|---|
| Citicoline | 300.000 | 49,180 |
| *Ginkgo biloba* dried extract | 40.000 | 6.557 |
| *Ginkgo biloba* dimeric flavones | 10.000 | 1.639 |
| Microcrystalline cellulose | 83.000 | 13.607 |
| Dicalcium phosphate | 50.000 | 8.197 |
| Silicon dioxide | 7.000 | 1.148 |
| Magnesium stearate | 10.000 | 1.639 |
| | | |
| *Gelatin shell* | *110* | |
| | | |
| **TOTAL** | **610.000** | |

### EXAMPLE 6 - 0+ format 610 mg capsules

| **INGREDIENT** | **mg/unit dose** | **%** |
|---|---|---|
| Citicoline | 300.000 | 49,180 |
| *Ginkgo biloba* dried extract phytosome | 40.000 | 6.557 |
| *Ginkgo biloba* dimeric flavones phytosome | 10.000 | 1.639 |
| Microcrystalline cellulose | 83.000 | 13.607 |
| Dicalcium phosphate | 50.000 | 8.197 |
| Silicon dioxide | 7.000 | 1.148 |
| Magnesium stearate | 10.000 | 1.639 |
| | | |
| *Gelatin shell* | *110* | |
| **TOTAL** | **610.000** | |

### EXAMPLE 7 - 750 mg swallowable tablets

| **INGREDIENT** | **mg/unit dose** | **%** |
|---|---|---|
| Citicoline | 400.000 | 53.333 |
| *Ginkgo biloba* dried extract | 40.000 | 5.333 |
| *Ginkgo biloba* dimeric flavones | 20.000 | 2.667 |
| Microcrystalline cellulose | 130.000 | 17.333 |
| Dicalcium phosphate | 119,000 | 15.867 |
| Sodium carboxymethylcellulose | 20.000 | 2.667 |
| Silicon dioxide | 7.000 | 0.933 |
| Magnesium stearate | 14.000 | 1.867 |
| | | |
| **TOTAL** | **750.000** | |

### EXAMPLE 8 - 1.5 g chewable tablets

| **INGREDIENT** | **mg/unit dose** | **%** |
|---|---|---|
| Citicoline | 400.000 | 26.667 |
| *Ginkgo biloba* dried extract | 40.000 | 2.667 |
| *Ginkgo biloba* dimeric flavones | 20.000 | 1.333 |
| Isomalt | 911.000 | 60.733 |
| Citric acid | 50.000 | 3.333 |
| Flavouring | 50.000 | 3.333 |
| Acesulfame K | 8.000 | 0.533 |
| Silicon dioxide | 7.000 | 0.467 |
| Magnesium stearate | 14.000 | 0.933 |
| | | |
| **TOTAL** | **1500.000** | |

### EXAMPLE 9 - 750 mg swallowable tablets

| **INGREDIENT** | **mg/unit dose** | **%** |
|---|---|---|
| Citicoline | 400.000 | 51.948 |
| *Ginkgo biloba* dried extract phytosome | 40.000 | 5.195 |
| *Ginkgo biloba* dimeric flavones phytosome | 20.000 | 2.597 |
| Microcrystalline cellulose | 150.000 | 19,481 |
| Dicalcium phosphate | 119,000 | 15.455 |
| Sodium carboxymethylcellulose | 20.000 | 2.597 |
| Silicon dioxide | 7.000 | 0.909 |
| Magnesium stearate | 14.000 | 1.818 |
| | | |
| **TOTAL** | **770.000** | |

### EXAMPLE 10 - 1.5 g chewable tablets

| **INGREDIENT** | **mg/unit dose** | **%** |
|---|---|---|
| Citicoline | 400.000 | 26.667 |
| *Ginkgo biloba* dried extract phytosome | 40.000 | 2.667 |
| *Ginkgo biloba* dimeric flavones phytosome | 20.000 | 1.333 |
| Isomalt | 911.000 | 60.733 |
| Citric acid | 50.000 | 3.333 |
| Flavouring | 50.000 | 3.333 |
| Acesulfame K | 8.000 | 0.533 |
| Silicon dioxide | 7.000 | 0.467 |
| Magnesium stearate | 14.000 | 0.933 |
| | | |
| **TOTAL** | **1500.000** | |

### EXAMPLE 11 - 1.3 g chewing gums

| **INGREDIENT** | **mg/unit dose** | **%** |
|---|---|---|
| Citicoline | 100.000 | 7.692 |
| *Ginkgo biloba* dried extract | 40.000 | 3.077 |
| *Ginkgo biloba* triterpenes | 10.000 | 0.769 |
| Sorbitol | 539,804 | 41.523 |
| Gum base | 310.000 | 23.846 |
| Isomalt | 250.463 | 19,266 |
| Silicon dioxide | 20.000 | 1.538 |
| Magnesium stearate | 20.000 | 1.538 |
| Flavouring | 6.454 | 0.496 |
| Acesulfame K | 1.640 | 0.126 |
| Aspartame | 1.640 | 0.126 |
| | | |
| **TOTAL** | **1300.000** | |

### EXAMPLE 12 - 1.3 g_ chewing gums

| **INGREDIENT** | **mg/unit dose** | **%** |
|---|---|---|
| Citicoline | 100.000 | 7.692 |
| *Ginkgo biloba* dried extract phytosome | 40.000 | 3.077 |
| *Ginkgo biloba* triterpenes phytosome | 10.000 | 0.769 |
| Sorbitol, | 499,804 | 38.446 |
| Gum base | 350.000 | 26.923 |
| Isomalt | 250.463 | 19,266 |
| Silicon dioxide | 20.000 | 1.538 |
| Magnesium stearate | 20.000 | 1.538 |
| Flavouring | 6.454 | 0.496 |
| Acesulfame K | 1.640 | 0.126 |
| Aspartame | 1.640 | 0.126 |
| **TOTAL** | **1300.000** | |

## Claims

1. Compositions containing citicoline, extracts of *Ginkgo biloba* leaves and dimeric flavones of *Ginkgo biloba* together with excipients and/or diluents.

2. Compositions as claimed in claim 1, containing dried extract of *Ginkgo biloba.*

3. Compositions as claimed in claim 1, containing extract of *Ginkgo biloba* or the active ingredients contained in it in the form of a complex with phospholipids.

4. Compositions as claimed in claim 1, containing dimeric flavones of *Ginkgo biloba* in the form of a complex with phospholipids.

5. Compositions as claimed in the preceding claims, wherein the doses of the active ingredients fall into the following weight intervals:
a) citicoline: between 10 and 1000 mg per unit dose;
b) dried extract of *Ginkgo biloba* or corresponding complex with phospholipids: between 10 and 360 mg per unit dose; and
c) dimeric flavones of *Ginkgo biloba* or corresponding complex with phospholipids: between 5 and 150 mg per unit dose.

6. Compositions as claimed in claim 5, wherein the doses of the active ingredients fall into the following weight intervals:
a) citicoline: between 100 and 400 mg per unit dose;
b) dried extract of *Ginkgo biloba* or corresponding complex with phospholipids: between 40 and 120 mg per unit dose; and
c) dimeric flavones of *Ginkgo biloba* or corresponding complex with phospholipids: between 10 and 60 mg per unit dose.

7. Compositions as claimed in any one of claims 1 to 6, also including additional ingredients selected from vitamins, aminoacids and trace elements.

8. Compositions as claimed in claim 7, wherein the additional ingredients are selected from L-arginine (or any other aminoacid or aminoacid-like substance able to promote the synthesis, release or metabolism of nitric oxide), selenium, zinc, magnesium, vitamin A, vitamin E, and group B vitamins.

9. Compositions as claimed in any one of claims 1 to 8, in the form of hard or soft gelatin capsules, tablets, effervescent or chewable tablets, granules or powders in sachets, solid controlled-release forms, and chewing gums.

10. Use of a combination of citicoline, a *Ginkgo biloba* extract, possibly complexed with phospholipids, and dimeric flavones of *Ginkgo biloba,* possibly complexed with phospholipids, for the preparation of a formulation for the treatment and prevention of vertigo and tinnitus.
